# EUROPEAN PATENT SPECIFICATION

(11) **EP 1 673 105 B1**
(45) Date of publication and mention of the grant of the patent: **02.05.2007**
(21) Application number: 04766306.7
(22) Date of filing: 23.07.2004
(51) Int. Cl.: A61K 38/24, A61P 15/00

(54) **USE OF FOLLICLE STIMULATING HORMONE FOR REDUCTION OF SPERMATOZOA CHROMOSOMAL ABERRATION IN MALES**
VERWENDUNG DES FOLLIKEL STIMULIERENDEN HORMONS ZUR REDUZIERUNG VON CHROMOSOMALEN ABERRATIONEN DER SPERMATOZOEN IN MANN
UTILISATION DE L'HORMONE DE STIMULATION FOLLICULAIRE POUR REDUIRE LES ABERRATIONS CHROMOSOMIQUES DES SPERMATOZOIDES MASCULINES

(30) Priority: 25.07.2003 EP 03102303; 26.02.2004 EP 04100760
(43) Date of publication of application: 28.06.2006
(73) Proprietor: LABORATOIRES SERONO S.A., 1170 Aubonne (CH)
(72) Inventor: DE LEO, Vincenzo, I-53100 Siena (IT); LA MARCA, Antonio, 41100 Modena (IT)
(74) Representative: Merck Serono International S.A. Intellectual Property
(86) International application number: PCT/EP2004/051593
(87) International publication number: WO 2005/011726

(56) References cited:
- US-A- 5 017 557
- ZION B-R ET AL: "Follicle-stimulating hormone treatment for men with idiopathic oligoteratoasthenozoospermia before in vitro fertilization: the impact on sperm microstructure and fertilization potential" FERTILITY AND STERILITY, ELSEVIER SCIENCE INC, NEW YORK, NY, US, vol. 73, no. 1, January 2000 (2000-01), pages 24-30, XP002275816 ISSN: 0015-0282
- BACCETTI B ET AL: "The effect of follicle stimulating hormone therapy on human sperm structure (Notulae seminologicae 11)" HUMAN REPRODUCTION, IRL PRESS, OXFORD, GB, vol. 12, no. 9, September 1997 (1997-09), pages 1955-1968, XP002275817 ISSN: 0268-1161 cited in the application
- STREHLER E ET AL: "THE EFFECT OF FOLLICLE-STIMULATING HORMONE THERAPY ON SPERM QUALITY: AN ULTRASTRUCTURAL MATHEMATICAL EVALUATION" JOURNAL OF ANDROLOGY, J.B. LIPPINCOTT CO., PHILADELPHIA, US, vol. 18, no. 4, July 1997 (1997-07), pages 439-447, XP008027039 ISSN: 0196-3635

## Description

### Field of the invention

This invention relates to the use of a substance selected from Follicle Stimulating Hormone (FSH) and FSH variants for the preparation of a pharmaceutical composition for reducing gamete chromosomal alterations in the male. More specifically, the invention is for use in men suffering from spermatozoa aneuploidy, notably diploidy and disomy. The invention further comprises the use of pharmaceutical formulations containing Follicle Stimulating Hormone (FSH) and FSH variants for the preparation of a pharmaceutical composition for the treatment and/or prevention of gamete chromosomal alterations in the male.

### Background of the invention

It has been shown that a considerable percentage of infertile men have an abnormal karyotype and, therefore, these subjects produce gametes with chromosomal alterations. Furthermore, having a normal karyotype does not exclude the possibility that spermatozoa with chromosomal alterations arc present, since errors in chromosomal segregation can occur during the mitotic and/or meiotic division in spermatogenesis. Based on the first data available in the literature (*Vegetti et al., 2000 Human Reproduction, 15(2), 351-365),* it has been suggested that approximately 0.3-1.08% of the spermatozoa of normal men have numerical chromosomal aberrations and that this percentage becomes higher when examining the spermatozoa of men with oligospermia (0.7-9.44%) and teratospermia (1.3-3.9%). These alterations appear to be related to the sex chromosomes and autosomes, particularly chromosomes 1, 18, and 21.
These sperm karyotype abnormalities generally result in reduced fertilization and/or increased abortion rate, i.e. fertile men producing gametes with chromosomal alterations have their female partner experiencing several miscarriage pregnancies *(Egozcue et al., 2003, Placenta, 24, S62-S65; Egozcue et al., 2000, Human reproduction, 6, 93-105*).

Numerical chromosomal aberration characterized by extra or missing chromosomes is called "aneuploidy".

Different types of aneuploidies are known and they are designated according to the kind of chromosomal numerical aberration: for example, the presence of one additional chromosome compared to the normal number in diploid cells is known as trisomy (2n +1); the absence of one chromosome in a homologous pair in diploid cells is known as monosomy (2n-1), while the loss of an entire chromosomal pair in diploid cells is known as nullisomy (2n-2), wherein n is the number of types of chromosomes.

By analogy, in the case of haploid cells, such as gametes, the term "aneuploid" characterizes gametes having a chromosomal numerical aberration, such as for example having an extra chromosome or missing one chromosome. These "aneuploid" gametes will form, when fused with a normal gamete, a zygote having an abnormal number of chromosomes (aneuploidy). Most of the time, the aneuploid zygotes die during the time between conception and birth. However, in some cases, the zygote becomes an offspring affected by "aneuploidy" and the consequences on the carrier of this chromosomal numerical aberration depends on the chromosomes which are involved.

The development of gametic aneuploidy of autosomes and sex chromosomes results from errors, such as failed separation of brother chromatids that occur during mitosis of spermatogonia and during the first and second meiotic division or such as failed separation of homologous chromosomes in mciosis. When the chromosomes fail to separate during the first meiotic phase, all the produced spermatozoa will have an aberrant number of chromosomes (only 2n:diploids or On). If the chromosomes fail to separate during the second meiotic phase, only 2 haploid cells out of the 4 coming from the same spermatocyte will be affected by chromosomal numerical aberration.

Therefore production of gamete aneuploidies constitutes a serious genetic risk factor as they induce aneuploidy in the offsprings which causes a lot of abnormalities in the foetus and in the viable infants.

For example, aneuploidy in the infant can cause infant later infertility, miscarriages and perinatal mortality of the infant, congenital malformations, mental retardation, and abnormal behavior *(Hook, 1985, in: Aneuploidy: Etiology and mechanisms. (eds) V.L*. *Dellarco, P.E. Voytek, A. Hollander, pp. 7-33. New York: Plenum; Hecht et al., 1987, in: Aneuploidy. Part A: Incidence and etiology. (eds) B.K. Vig, A.A. Sandberg, 9-49. New York: Alan R. Liss),* increased sensitivity to infectious diseases, high propensity of leukeamia or early development of Alzheimer disease in the offspring.

Aneuploidies in autosomes such as monosomies are always lethal for the foetus whereas trisomies are non lethal only when they involve chromosomes 13, 18 and 21 but are dramatically handicapping for the offspring.

Examples of common sex chromosome aneuploidy are found in patients with Klinefelter Syndrome (47, XXY) which is the most common form of aneuploidy in men and in patients (47, XYY) which is another form of aneuploidy discovered by Sandberg and co-wokers.

The presence of spermatozoa chromosomal abnormalities (both numerical and structural) has been noted in a considerable percentage of infertile patients *(Bourrouillou et al., 1985, Hum Genet, 71, 366-367)* and it is widely accepted that using assisted fertilization techniques allows these patients to procreate, increasing the risk of having children with chromosomal alterations.

Aneuploid gametes also develop in subjects with oligoasthenoteratozoospermia (OAT) with normal chromosomal sets since noxious testicular pathogens can disrupt the delicate process of chromosomal segregation during spermatogenesis. Aneuploid gametes can also develop in older subjects or in patients exposed to potentially ancuploidogenic agents. For this reason, evaluating the frequency of aneuploidy in spermatozoa is becoming a fundamental stage in the diagnostic course of infertile patients, particularly if they intend to undergo assisted fertilization techniques.

Published data indicate that patients with OAT have an increased percentage of spermatozoa with hereditary chromosomal aneuploidy. This increased frequency of aneuploidy reduces the fertilizing capability of spermatozoa during assisted fertilization techniques (*Storeng et al., 1998, Acta Obstet Gynecol Scand, 77(2), 191-197).*

Considering the fact that spermatozoa separation methods used during fertilization in *vitro* and embryo transfer (FIVET) do not modify the percentage of aneuploid sperm, patients with OAT are at great risk of producing children with chromosomal aneuploidy (*Pfeffer et al., 1999, Fertil Steril, 72, 472-478*).

The development of fertilization techniques such as *in vitro* fertilization (IVF) has increased the importance of monitoring the sperm karyotype abnormalities as they can be the cause of the fertility disorders and/or can lead to induce chromosomal aberrations in the offsprings *(Levron et al., 2001, Molecular and Cellular Endocrinology, 183, S23 - S28*).

Male patients undergoing IVF are generally partner of infertile women because of tubal factor, anovulation or idiopathic infertility or male having a low quantity of spermataozoa.
Research in this area has become more clinically relevant in the past few years with the development of intra-cytoplasmic sperm injection (ICSI). ICSI has been shown to be extremely useful for the treatment of infertility, however transmission of cytogenic defects to offspring is a major concern with this fertilization technique and has been observed (*Ushijima et al., 2000, Human Reproduction, 15(5), 1107-1111; Levron et al., 2001, above*). The risk is high for example for sperm of donors with high degrees of consanguinity or translocation *(Baccetti et al., 2002, Contraception, 65, 283-287).* Male patients undergoing ICSI are usually patients with mild or severe oligo-and/or terato- and/or astheno-zoospermia. Moreover, sperm samples obtained by surgical procedures (Testicular Sperm Aspiration: TESA; Testicular Sperm Extraction: TESE; Microsurgical Epididymal Sperm Aspiration: MESA; Percutaneous Epididymal Sperm Aspiration: PESA) are also treated by ICSI.
For this reason, spermatic aneuploidy should be assessed in all patients who intend to undergo assisted fertilization techniques.

It would be desirable to develop methods for reducing the rate of aneuploidy such as diploidy in spermatozoa, especially in men who intend to undergo Assisted Reproduction Techniques (ART).

Follicle-stimulating hormone (FSH) is known for its role in the initial development of Sertoli cells and in their stimulation for controlling spermatogonia. It is suggested by experimental studies that FSH is present in Sertoli cells and in round germinal cells that both express the FSH receptor *(Baccetti et al., 1998, Human Reproduction, 12, 9, 1955-1968).*

Furthermore, exogenous FSH therapies induced improvements in the sperm morphology and ultrastructure in men having alterations in acrosome, chromatin and axonemes as visualized through electron microscopy *(Baccetti et al., 1997, The FASEB Journal, 12,1045-1054)* and in men with idiopathic oligoasthenozoospermia *(Ben-Rafael, 2000, Fertility and Sterility, 73 (1), 24-30; Strehler et al., 1997, Journal of Andrology, 18(4), 439-447).*

FSH is used to induce spermatogenesis in men suffering from oligospermia. A regimen using 150 IU FSH, 3 times weekly in combination with 2'500 IU hCG (human Chorionic Gonadotrophin) twice weekly has been successful in achieving an improvement in sperm count in men suffering from hypogonadotrophic hypogonadism (*Burgues et al., 1997, Hum. Reprod., 12, 980-6*). High dose FSH (150 IU) has been used to treat iodopathic oligospermia (*Iacono et al., 1996, J. Urol., 102, 81-4*).

These studies are focused on the increase in the fertility rate and improvements of sperm morphology and ultrastructure as visualized by Scanning Electron Microscopy (SEM)/Transmission Electron Microscopy (TEM).
Aneuploidy does not affect karyoplasms when observed by SEM/TEM and does not correlate with SEM/TEM characteristics, i.e. semen with normal characteristics in SEM/TEM can be affected by aneuploidy. *Bacetti et al., 1997, above,* suggest that patients with genetic defects should not be treated with FSH.

### Summary of the invention

It is an object of the invention to provide a method for the reduction and/or prevention of gamete chromosomal alterations in a male, notably spermatozoa aneuploidy, including diploidy and disomy, preferably in men intending to undergo assisted fertilization techniques, whereby a pharmaceutically active amount of FSH or FSH variant is administered to the male in need thereof.

In a first aspect, the invention provides the use of a substance selected from Follicle Stimulating Hormone (FSH) and FSH variants for the preparation of a pharmaceutical composition for the treatment and/or the reduction of gamete chromosomal alterations in a male, notably spermatozoa aneuploidy, including diploidy and disomy.

The invention is useful in a method for reducing gamete numerical chromosomal alterations in a male subject, notably spermatozoa aneuploidy, including diploidy and disomy, comprising administering an effective dose of a substance selected from FSH and a FSH variant to the patient.

The invention is useful in a method for preventing the occurrence of chromosomal aberrations in the offspring of a male subject, comprising administering an effective dose of a substance selected from FSH and a FSH variant to the male subject prior to conception.

### Detailed description of the invention

The following paragraphs provide definitions of various terms, and are intended to apply uniformly throughout the specification and claims unless an otherwise expressly set out definition provides a different definition.

"Oligozoospermia" is characterized by a semen containing a low sperm count. It is usually diagnosed in men when sperm concentration <20 x 10⁶/ml.

"Asthenozoospermia" is characterized by a semen having an impaired sperm motility. It is usually diagnosed in men when fewer than 50% spermatozoa have forward progression or fewer than 25% spermatozoa have rapid linear progression.

"Teratozoospermia" by a semen having sperm with an abnormal morphology. It is usually diagnosed in men when fewer than 30% spermatozoa have normal morphology.

"Oligoasthenoteratozoospermia" is characterized by a semen having all the three previously cited variables for sperm quality, which are disturbed. According to WHO criteria, it is diagnosed when patients display the following parameters: density <20 × 10⁶ spermatozoa/mL, motility of grade 3 <25% and/or motility of grade 2 plus 3 <50%, and normal morphology in less than 30% of spermatozoa.

The term "karyotype" refers to the chromosomal constitution of an individual, including the number of chromosomes and any possible abnormalities.

Exemplary diseases where chromosomal aberrations are involved include Down's syndrome, Klinefelter's syndrome (XXY), Turner's syndrome (XO), Triplo-X syndrome, Tetra-X syndrome, Penta-X syndrome, XYY syndrome, monosomies or polysomies of any chromosome including 7, 10, 11, 13, 18, 21, X and Y, sex chromosome aneuploidy in men with chromosomal mosaicism (XY/XXY) and multiple X chromosomes (e.g. XXXXY; XXXYY).

The term "administer" or "administering" means to introduce a formulation of the present invention into the body of a patient in need thereof to treat a disease or condition.

The term "chromosomal aberration" is used for chromosomal abnormalities and includes chromosomal numerical aberrations such as polyploidy and aneuploidy which includes the presence of at least an extra chromosome or the absence of one chromosome. Aneuploidy in haploid cells such as gametes includes diploidy.

"Ancuploidy" and "polyploidy" are conditions in which a cell has a number of chromosomes different from the usual haploid number ("n") or diploid number ("2n").

As it is known, in higher plants and animals, each specific type of chromosome is normally present as two copies of homologous chromosomes (diploid state). For example, in humans, the diploid number is 46.

After conclusion of the two cell divisions that form the sex cells, i.e. the gametes (the sperm and the egg), each resulting sperm and egg usually enclose only one copy of each chromosomes (haploid state). For example, in humans, the normal haploid number (number of chromosome in haploid cells such as gametes) is 23.

Union of sperm and egg during fertilization results in a "zygote" containing one homologous chromosome from the male parent and another from the female parent, the normal diploid chromosome constitution, being restored.

A normal somatic cell should have a diploid chromosome content of 23 pairs, or 46 chromosomes in total.
Therefore, an aneuploid somatic cell has a number of chromosomes that is other than twice the normal haploid number (2n). For example, an aneuploid somatic cell can be a cell having a trisomy, i.e., a cell having three copies of one chromosome, or a monosomy, i.e., a cell having a single copy of one chromosome. Aneuploidy can result from chromosomal non-disjunction during mitosis. A polyploid cell is a cell having a number of chromosomes that is some multiple of the normal haploid number greater than the usual diploid number, i.e., 2n. For example, a polyploid cell can be triploid (n=69) or tetraploid (n=92).

In a haploid cell, such as a gamete, the natural state is haploid, meaning n chromosomes. Aneuploidy means that the cell has a chromosome number that is other than n. For example, one chromosome may be missing (n-1), or an extra chromosome may be added (n+1) such as in disomy, or the number of chromosomes can be doubled such as in diploidy (2n).

"Disomy" in cells is characterized by the presence of an extra chromosome chromosome. In haploid cells, such as gametes, disomy is characterized by n+1 chromosomes.

"Diploidy" in cells is characterized by the doubling of the number of chromosomes. In haploid cells, such as gametes, diploidy is characterized by 2n chromosomes.

The term "patient" means a male mammal that is treated for a disease or condition. Patients are of, but not limited to, the following origin, human, ovine, porcine, equine, bovine, rabbit and the like.

The term "Assisted Reproduction Technology" includes IVF (In Vitro Fertilization), ICSI (intra-cytoplasmic sperm injection).

By "effective amount", is meant an amount of FSH sufficient to reduce gamete chromosomal alterations in a male subject, especially to reduce the rate of aneuploidy, including diploidy and disomy in a male patient. The amount of FSH can be routinely determined by those of skill in the art. The amount of the compound actually administered will typically be determined by a physician, in the light of the relevant circumstances, including the condition to be treated, the chosen route of administration, the actual compound administered, the age, weight, and response of the individual patient, the patient's endogenous FSH levels, and the like. The "effective amount" can alternatively be achieved by the duration of the treatment. Typically, the duration of the treatment includes treatments of at least one month, three month, six months or one year.

The expression "pharmaceutically acceptable" is meant to encompass any carrier or salt which docs not substantially interfere with the effectiveness of the biological activity of the active ingredient and that is not toxic to the host to which is administered.

The term "recombinant" refers to preparations of FSH or FSH variants that are produced through the use of recombinant DNA technology.

The rate of aneuploidy, diploidy or disomy can be determined through the evaluation of numerical chromosomal abnormalities in ejaculated spermatozoa for example by fluorescence *in situ* hybridisation (FISH), especially multicolour FISH *(Egozcue et al., 2003, above).* FISH involves hybridisation of specific DNA probes labelled with fluorochromes to complementary DNA sequences on target chromosomes, followed by detection of the bound probes under a fluorescence microscope as described in *Shi et al., 2001, Reproduction, 121, 655-666.* To perform FISH analysis on semen samples from donors with extremely low quantities of sperm, a microwave decondensation/codenaturation technique has been developed to increase analyzable sperm numbers for use in fluorescence *in situ* hybridization (FISH) (*Rademaker et al., 2001, Cytogenet Cell Genet. 95(3-4), 143-5*). Example of a FISH protocol for determining the rate of aneuploidy or diploidy is detailed in Example n° 1.

The levels of basal serum testosterone can be measured by commercially available ELISA/chemiluminescent immunoassays.

The expression "FSH variant" is meant to encompass those molecules differing in amino acid sequence, glycosylation pattern or in inter-subunit linkage from human FSH but exhibiting FSH-activity. Examples include CTP-FSH, a long-acting modified recombinant FSH, consisting of the wild type α-subunit and a hybrid β-subunit in which the carboxy terminal peptide of hCG has been fused to the C-terminal of the β-subunit of FSH, as described in *(La Polt et al., 1992, Endocrinology, 131, 2514-2520* or *Klein et al., 2003, Human Reprod. 2003, 18, 50-56*). Also included is single chain CTP-FSH, a single chain molecule, consisting of the following sequences (from N-terminal to C-terminal):

| | | |
|---|---|---|
| βFSH | βhCG-CTP(113-145) | αFSH |

wherein βFSH signifies the β-subunit of FSH, βhCG CTP (113-145) signifies the carboxy terminal peptide of hCG and αFSH signifies the α-subunit of FSH, as described by Klein et al., 2003. Other examples of FSH variants include FSH molecules having additional glycosylation sites incorporated in the α- and/or β-subunit, as disclosed in WO 01/58493, particularly as disclosed in claims 10 and 11 and FSH molecules with inter-subunit S-S bonds, as disclosed in WO 98/58957.

The FSH variants referred to herein also include the carboxy terminal deletions of the beta subunit that are shorter than the full length mature protein.

The FSH used in the invention can be "recombinant" or from a natural source.

The FSH variants used in the invention can be "recombinant" or obtained by chemical synthesis.

An example of recombinant DNA technology for producing FSH is described in WO 85/01958.

One example of a method of expressing FSH using recombinant technology is by transfection of eukaryotic cells with the DNA sequences encoding an alpha and beta subunit of FSH, whether provided on one vector or on two vectors with each subunit having a separate promoter, as described in European patent nos. EP0211894 and EP0487512.

Another example of the use of recombinant technology to produce FSH is by the use of homologous recombination to insert a heterologous regulatory segment in operative connection to endogenous sequences encoding one or both of the subunits of FSH, as described in European patent no. EP0505500.

Also contemplated arc methods such as those disclosed in WO99/57263, wherein one of the subunits is inserted heterologously into a cell, and the other subunit is expressed by activation of genomic sequences by insertion of a heterologous regulatory segment by homologous recombination. The invention may be used with any of these methods of expressing FSH.

Recombinant human FSH that is suitable for use in the invention is, for example, GONAL-f^{™} (FSH, Follitropin alpha) from SERONO.

The FSH used in accordance with the present invention may be purified from biological sources, such as from urinary sources, e.g. urinary FSH or urofollitropin (uFSH) . Acceptable methodologies include those described in *Hakola, 1997, Molecular and Cellular Endocrinology, 127:59-69; Keene et al., 1989, J. Biol. Chem., 264:4769-4775; Cerpa-Poljak et al., 1993, Endocrinology, 132:351-356; Dias et al. 1994, J. Biol. Chem., 269:25289-25294; Flack et al., 1994, J. Biol. Chem., 269:14015-14020* and *Valove, et al., 1994, Endocrinology, 135:2657-2661;* U.S. Patent 3,119,740 and US Patent no. 5,767,067.

FSH or a FSH variant may be formulated for injection, either intra-muscular or subcutaneous, preferably subcutaneous.

The FSH formulation may be freeze-dried, in which case it is dissolved in water for injection just prior to injection. The FSH formulation may also be a liquid formulation, in which case it can be injected directly, without prior dissolution.

The FSH formulation may be in both single dose or multiple dose liquid formats, in vials, or ampoules. Single dose formats must remain stable and potent in storage prior to use. Multi-dose formats must not only remain stable and potent in storage prior to use, but must also remain stable, potent and relatively free of bacteria over the multiple use regimen administration period, after the seal of the ampoule has been compromised. For this reason, multi-dose formats often contain a bacteriostatic agent, such as, for example, benzyl alcohol, meta-cresol, thymol or phenol, preferably benzyl alcohol or meta-cresol. Single dose formulations may also comprise a bacteriostatic agent.

FSH or FSH variants may be formulated with known excipients and stabilizers, for example, sucrose and mannitol. The formulation may also comprise an antioxidant, such as methionine. It may further comprise a surfactant, such as TWEEN (preferably TWEEN 20) or Pluronic (preferably Pluronic F68).

FSH is currently formulated for intra-muscular (IM) or subcutaneous (SC) injection. It is supplied in a lyophilised (solid) form in vials or ampoules of 75 IU/vial and 150 IU/vial with a shelf life of one and a half to two years when stored at 2-25°C. A solution for injection is formed by reconstituting the lyophilised product with water for injection (WFI). Depending on the patient's response, up to three cycles of treatment with increasing doses of FSH can be used. With lyophilised formulations, the patient is required to reconstitute a new vial of lyophilised material with diluent and administer it immediately after reconstitution on a daily basis: for example: [Package insert N1700101A, published in February 1996, for Fertinex^{™} (purified urofollitropin for injection) for subcutaneous injection, by Serono Laboratories, Inc., Randolph, MA] ; Metrodin HP®; Gonal F® pen (Serono Laboratories, Inc), i.e. pre-filled and ready-to-use multi-dose FSH.

### Examples of formulations for FSH are listed below:

EP0618808 (Applied Research Systems ARS Holding N.V.) discloses a pharmaceutical composition comprising a solid intimate mixture of gonadotrophin and a stabilising amount of sucrose alone or in combination with glycine.

PCT/EP2004/050432 (Applied Research Systems ARS Holding N.V.) discloses a pharmaceutical composition of FSH and Pluronic.

EP0448146 (AKZO N.V.) discloses a stabilized gonadotrophin containing lyophilisate comprising one part by weight of a gonadotrophin; and 200 to 10,000 parts by weight of a dicarboxylic acid salt stabilizer associated with the gonadotrophin.

EP0853945 (Akzo Nobel N.V.) discloses a liquid gonadotrophin-containing formulation characterised in that the formulation comprises a gonadotrophin, stabilising amounts of a polycarboxylic acid or a salt thereof and of a thioether compound.

The levels of FSH may be increased in a male suffering from gamete chromosomal alterations, by raising endogenous FSH levels through the administration of FSH agonists, for example the FSH agonists described in WO 0209706 and WO 0008015.

In one embodiment, the invention provides the use a substance selected from Follicle Stimulating Hormone (FSH) and FSH variants for the preparation of a pharmaceutical composition for the treatment and/or the reduction of gamete chromosomal alterations in a male, notably numerical aberrations in spermatozoa such as gamete aneuploidy, including diploidy and disomy.

The invention is useful in a method for treating and/or preventing a disease or disorder associated with gamete chromosomal alterations in a male, notably numerical aberrations in spermatozoa such as gamete aneuploidy, including diploidy and disomy, comprising administering to a male subject in need thereof an effective amount of a substance selected from FSH and FSH variants, wherein the subject can be human or animal.

The invention is useful in a method for reducing gamete chromosomal alterations in a male, notably numerical aberrations in spermatozoa such as gamete ancuploidy, including diploidy and disomy, comprising administering an effective dose of a substance selected from FSH and FSH variants to the patient.

The invention is useful in a method for preventing the occurrence of chromosomal aberrations in the offspring of a male subject, comprising administering an effective dose of a substance selected from FSH and FSH variants to the male, prior to conception.

The invention is useful in a method of in *vitro* fertilization, including ICSI, comprising the step of treating the donor of spermatozoa, prior collection of spermatozoa, with an amount of FSH or FSH variant sufficient to prevent or reduce chromosomal aberrations in the spermatozoa.

The invention is useful in a method of *in vitro* fertilization, including ICSI, comprising the step of detecting aneuploidy and/or diploidy in the sperm of a patient by a technique such as Fluoresencent *in situ* hybridisation (FISH), treating the donor of aneuploid and/or diploid spermatozoa prior collection of spermatozoa, with an amount of FSH or FSH variant sufficient to prevent or reduce chromosomal aberrations in the spermatozoa.

In a preferred embodiment of the invention, the male is human.

In another preferred embodiment of the invention, the substance is FSH.

In another preferred embodiment of the invention, the substance is rFSH.

In another preferred embodiment of the invention, the male presents a numerical chromosomal aberration in spermatozoa.

In another preferred embodiment of the invention, the numerical chromosomal aberration in spermatozoa is diploidy.

In another preferred embodiment of the invention, the numerical chromosomal aberration in spermatozoa is sex chromosome disomy.

In another preferred embodiment of the invention, the male presents an aneuploidy in spermatozoa at least or about 1%.

In another preferred embodiment of the invention, the male presents a diploidy in spermatozoa of at least or about 0.5%.

Another preferred group of patient is represented by males presenting a diploidy of at least or about 0.8%.

Another preferred group of patient is represented by males presenting an aneuploidy and a basal serum level of total testosterone of at least or about 5,000 pg/ml.

In another preferred embodiment of the invention, the administration of the substance is performed on alternate days.

In another preferred embodiment of the invention, the substance is administered at or about 75 to 300 IU/dose, preferably at 150 IU/dose.

In another preferred embodiment of the invention, the male patient will undergo Assisted Reproduction Technology such as IVF (*in vitro* fertilization) and ICSI (intracytoplasmic sperm injection).

In another preferred embodiment of the invention, the treatment of the male patient with a substance selected from FSH and FSH variants, is performed during at least a time selected from one week, one month, three months, six months and one year, preferably at least three months before undergoing Assisted Reproduction Technology.

In one more preferred embodiment, the invention provides a use of rFSH for the preparation of a pharmaceutical composition for the treatment and/or the reduction of spermatozoa aneuploidy in a patient.

The invention is useful in a method for reducing spermatozoa aneuploidy in a patient, comprising administering an effective dose of a substance selected from rFSH to the patient.

The invention will now be described by means of the following Examples, which should not be construed as, in any way, limiting the present invention. The Examples will refer to the Figures specified here below.

### Description of the figures:

**Figure 1** represents the variation of the percentages of total aneuploidy at the different stages of the treatment, i.e. 150 UI/day on alternate days (-45: 45 days before the treatment; 0: the day of the treatment before the treatment; 90: 90 days after the beginning of the treatment).
**Figure 2** represents the variation of the percentages of diploidy respectively at the different stages of the treatment, i.e. 150 UI/day on alternate days (-45: 45 days before the treatment; 0: the day of the treatment before the treatment; 90: 90 days after the beginning of the treatment).
**Figure 3** represents the variation of the percentage of total disomy at the different stages of the treatment, i.e. 150 UI/day on alternate days (-45: 45 days before the treatment; 0: the day of the treatment before the treatment; 90: 90 days after the beginning of the treatment).

### Abbreviations:

**Kg** (kilogram), **mg** (milligram), **min** (minute), **ml** (milliliter), **mM** (millimolar), **m²** (square meter), **ng** (nanogram), **pg** (picogram)
**ART** (Assisted Reproduction Technique), **BMI** (Body mass index), **CEP** (Chromosome Enumeration Probes), **DAPI** (4,6 Diamidino-2-phenylindole), **DHEA** (Dehydro epiandrosterone), **DTT** (dithiothreitol, Biorad), **EDTA** (ethylenediaminetetraacetic acid), **FIVET** (Fertilization *In Vitro* and Embryo Transfer), **FISH** (Fluorescent *in situ* Hybridization), **FSH** (Follicle Stimulating Hormone), **hCG** (human Chorionic Gonadotropin), **ICSI** (Intracytoplasmic Sperm Injection), **IM** (intra-muscular), **IVF** (*In Vitro* Fertilization), **IU** (International Unit), **LIS** (3,5 di-iodosalicylic acid lithium salt), **MESA** (Microsurgical Epididymal Sperm Aspiration), **OAT** (oligoastheno teratozoospermia), **PESA** (Percutaneous Epididymal Sperm Aspiration), **rFSH** (Recombinant FSH), **RIA** (Radioimmuno-assay), **RT** (room temperature), **SEM** (Scanning Electron Microscopy), **SC** (subcutaneous), **SCC** (sodium chloride/sodium citrate), **TEM** (Transmission Electron Microscopy), **TESA** (Testicular Sperm Aspiration), **TESE** (Testicular Sperm Extraction), **TSH** (Thyroid Stimulating Hormone; **uFSH** (urinary FSH), **USP** (United States Pharmacopeia), **WFI** (Water For Injection).

### EXAMPLES

The invention will be illustrated by means of the following examples which are not to be construed as limiting the scope of the invention.

### EXAMPLE 1: Effect of GONAL-f^{™} (recombinant hFSH from SERONO) on aneuploidy in spermatozoa of human infertile males (8 patients).

### a) Selection of infertile patients

Infertility of patients is determined after an andrological visit comprising spermiogram record, hormonal measurement (Androstenedione (A), total testosterone (Ttot), DHEAs, Prolactine (PRL), TSH, free tyroxine (FT4)) and microdeletion of chromosome Y.

### Normal values are the following:

**A:** 1200-5000 ng/ml); **T tot:** 1500-11400 pg/ml; **PRL:** 2-15 ng/ml; **FT4:** 50-120 ng/ml; **FSH:** 0.7 - 10 mIU/ml.
Blood samples are obtained between 9-11 a.m. Measurements are performed by commercial RIA or ELISA kits.

### b) Aneuploidy/diploidy rates in sex chromosomes determination by FISH analysis before treatment

Fresh sperm samples were washed with 150 mM NaCl and 10 mM Tris-HCl (pH 8), smeared on glass slides and dried in air. They were then fixed in 3:1 methanol-acetic acid for 10 min. The slides were dehydrated in 70%, 80% and 100% cold ethanol and air dried. Samples were swollen treated with 0.01 M DTT (dithiothreitol, Biorad) in 0.1M Tris-HCl (pH 8) and then 20 mM LIS (3,5 di-iodosalicylic acid lithium salt, Sigma) in the same buffer, checking sperm head swelling. The slides, rinsed in 2X SSC (pH 7) and air dried, were dehydrated and denatured in 70% formamide (Aldrich) 2X SSC at 73° for 4 min. They were then quickly dehydrated in a graded ethanol series at 0°C and air dried.

During this last step, CEP (Chromosome Enumeration Probes, Vysis, IL, USA) - satellite DNA probes for chromosomes X, Y and 18 directly labelled with different fluorochromes were used. The probe mix was denatured for 5 min at 73°C in a water bath. Hybridization was carried out at 37°C in a moist chamber for 12 hours. The slides were then washed with 0.4X SSC-0.3% NP40 for 2 min at 73°C, quickly in 2X SSC-0.1% NP40 at RT and finally mounted with DAPI 125 ng/ml in antifade solution (Vysis, IL, USA). A total of 3500-5000 spermatozoa were analyzed by triple color FISH.

### SCORING CRITERIA

The overall hybridization efficiency was >99%. Sperm nuclei were scored according to criteria of *Martin et al. 1995, Mol. Reprod. Dev., 42(1): 89-93.* Sperm nuclei are scored only if they are intact, non-overlapping and have a clearly defined border.

In the case of aneuploidy, the presence of sperm tail was confirmed. A sperm was considered disomic if the two fluorescent spots are of same colour, comparable in size, shape and intensity and positioned inside the edge of the sperm head at least one domains apart.

Diploidy was recognized by the presence of two double fluorescent spots, following the above criteria. Observation and scoring was performed on a Leitz Aristoplan Optic Microscope equipped with fluorescence apparatus, with a triple band-pass filter for Aqua, Orange, Green Fluorocromes (Vysis, IL, USA) and a monochrome filter for DAPI.
Patients having the following characteristics are selected:
Diploidy represented the 50.24% of total aneuploidy observed before treatment.
   - Positive test for the presence of diploidy ≥ 0.8% by FISH measurement on ejaculated spermatozoa as described above.
   - FSH serum levels < 10 UI/ml.
Patients presenting at least one of these characteristics are excluded from the study:
   - Body mass index [(BMI = weight (kg)/height (m²) x 100] <30;
   - Presence of infection in the spermatic passageways (i.e. chlamydia, ureaplasma, mycoplasma) at baseline;
   - Known autoimmune disorders;
   - Thyroid disorders and/or pathologies;
   - Chronic hepatopathies;
   - Presence of concomitant pathologic conditions that arc contraindicated in FSH treatment.

### c) Treatment protocol:

Patients are administered with a dose equal to 150 UI (2 vials s.c.) on alternate days for 3 months.

### d) Aneuploidy/diploidy rates determination by FISH analysis after treatment

The rates of aneuploidy and diploidy in sex chromosomes are measured by FISH analysis after treatment following the protocol detailed above (b).

A reduction in diploidy by -54% was found on a sample size of 8 consecutive patients (95% confidence interval: -20% to -87%; n=3; p=0.02).

A reduction in the frequency of sex chromosome aneuploidy was found on a sample size of 8 consecutive patients.

### EXAMPLE 2: Effect of GONAL-f^{™} on diploidy and aneuploidy in 19 human infertile males.

### a) Patient selection:

A total of 23 infertile male patients from age 18 to 55 arc selected. Their infertility is determined according to the protocol detailed in Example 1.

### b) Treatment:

19 patients arc administered with a dose equal to 150 UI (2 vials s.c.) on alternate days for 3 months and 4 patients arc not treated.

The percentages of total aneuploidy, diploidy and total disomy is measured by the FISH test (see Example 1) 45 days before the treatment (-45), on the day of treatment before the treatment (0).
Total aneuploidy is the calculated as the sum of diploidy and total disomy. The percentages of specific disomies (sex chromosomes disomy and chromosome 18 disomy) are calculated. Sex chromosome disomy is further analysed into detailed percentages of disomy for chromosomes XX, YY and XY.

### c) End-point measurements:

After 3 months of treatment (90), the patients undergo the same examinations that are performed at the beginning of the study and the FISH tests are repeated to measure each aneuploidy, diploidy and disomy parameters.

### d) Statistical analysis of the measurements:

The statistical significances of differences between averages are calculated using the protocol detailed in Example 1.

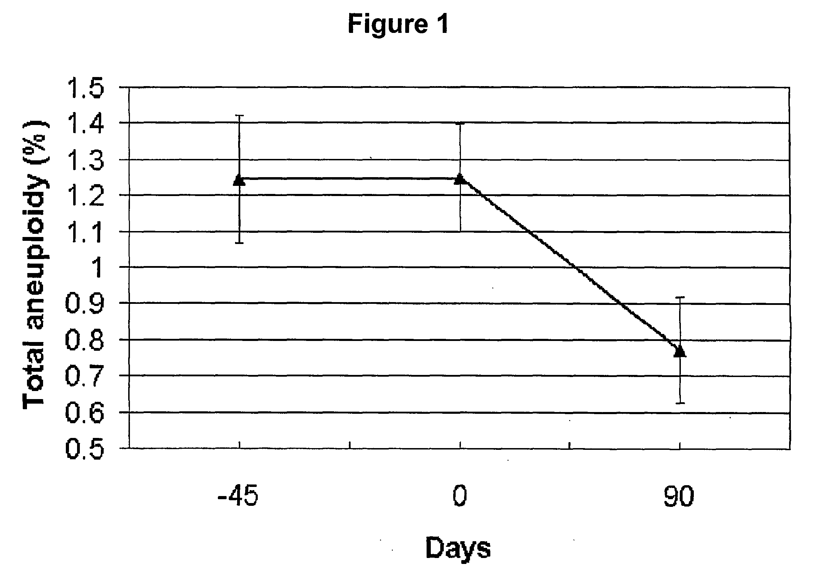

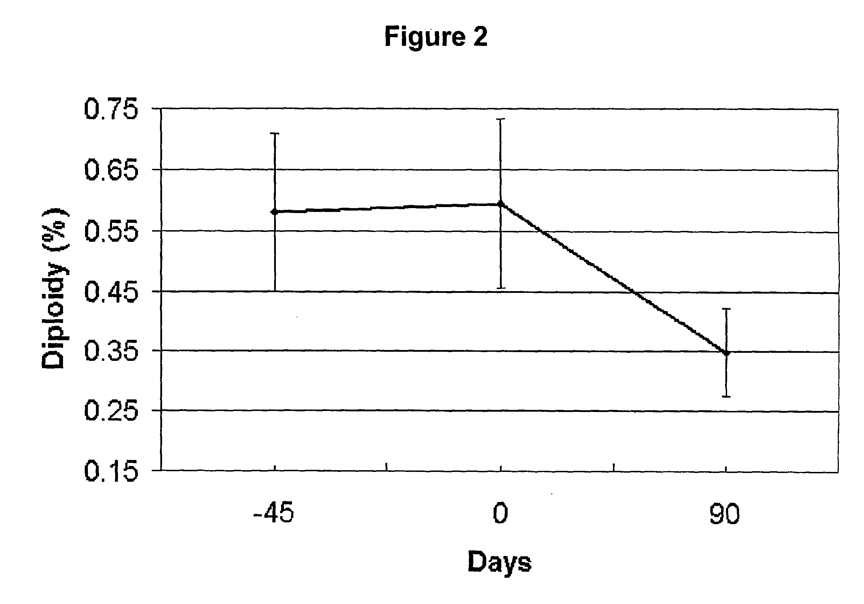

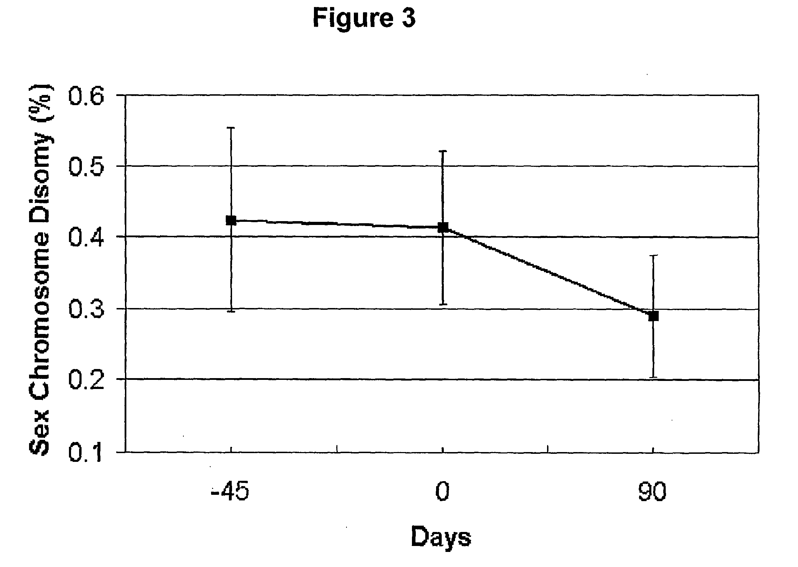

The effects of the 3-month-treatment with GONAL-f^{™} on total ancuploidy and diploidy are reported respectively on Figures 1 and 2.
A reduction of 38.4% in total aneuploidy and a reduction of 40.7% in diploidy are observed.
The effect of the 3-month-treatment with GONAL-f^{™} on total disomy is reported in Figure 3. The major reduction effect on total disomy observed after the treatment in due to the reduction of total sexual disomy (-29.3%). The major contribution to the reduction in total sexual disomy is found to be due to a reduction in the percentage of XX disomy (-35.5%) and YY disomy (-50%).

These results show that a treatment with recombinant FSH in men is able to reduce significantly total aneuploidy, especially diploidy and sex chromosome disomy.

### EXAMPLE 3: Effect of GONAL-f^{™} on diploidy and aneuploidy in 22 human infertile males.

The statistical data for 3 more patients that were selected in Example 2 but that were not included in the calculations of Example 2 have been analysed.

The effects shown on Example 2 on total aneuploidy, diploidy and sexual disomy are confirmed for the extended patient sample (22 patients): a 32% reduction in total aneuploidy, 33% reduction in diploidy and 26% reduction in total sexual disomy are observed.
The major contribution to the reduction in total sexual disomy is found to be due to a reduction in the percentage of XX disomy (-32%) and XY disomy (-21 %).

### EXAMPLE 4: Effect of GONAL-f^{™} on diploidy and aneuploidy in 6 human infertile males.

The protocol used was the same as the one used in Example 2, except that the administered dose of FSH was 300 IU on alternate days and the number of patients enrolled was 6.

The analysis of variables after 90 days of treatment was performed as described in Example and showed also a decrease in chromosomal aberrations (aneuploidy, diploidy, disomy of total sex chromosomes, disomy of XY, YY, XX chromosomes).

For statistical significance, the results of the 6 patients with the higher (300 IU) were cumulated with those of the study on the 22 patients receiving the lower dose (150 IU dose from Example 3).

This cumulated analysis resulted in the following conclusions: FSH treatment led to a 30% reduction in total aneuploidy, 32% reduction in diploidy, 28% reduction in total sexual disomy are observed, 23% reduction in disomy of XY chromosomes and 30% reduction in disomy of XX chromosomes.

Therefore, the increase of the number of patients, while increasing statistical significance confirmed the beneficial effect of FSH treatment in male aneuploidy.

## Claims

1. Use of a substance selected from Follicle Stimulating Hormone (FSH) and FSH variant for the preparation of a pharmaceutical composition for the treatment and/or the reduction of gamete chromosomal alterations in a male.

2. Use according to claim 1 wherein the gamete chromosomal alterations are numerical alterations.

3. Use according to claims 1 or 2 wherein the gamete numerical chromosomal alterations is spermatozoa diploidy.

4. Use according to any of the preceding claims wherein the gamete numerical chromosomal alterations is sexual chromosome disomy.

5. Use according to any of the preceding claims wherein the male is human.

6. Use according to any of the preceding claims wherein the substance is FSH.

7. Use according to any of the preceding claims wherein the substance is rFSH.

8. Use according to any of the preceding claims wherein the substance is administered on alternate days.

9. Use according to any of the preceding claims wherein the substance is administered at or about 75 to 300 IU/dose.

10. Use according to any of the preceding claims wherein the substance is administered at or about 150 IU/dose.

11. Use according to any of the preceding claims wherein the substance is rFSH and the human male is suffering from gamete ancuploidy.

## Patentansprüche

1. Verwendung einer Substanz, die ausgewählt ist aus follikelstimulierendem Hormon (FSH) und einer Variante von FSH, zur Herstellung einer pharmazeutischen Zusammensetzung zur Behandlung und/oder Reduzierung von chromosomalen Veränderungen von Gameten bei einem männlichen Lebewesen.

2. Verwendung gemäß Anspruch 1, wobei die chromosomalen Veränderungen von Gameten numerische Veränderungen sind.

3. Verwendung gemäß Ansprüchen 1 oder 2, wobei es sich bei den numerischen chromosomalen Veränderungen von Gameten um Diploidie von Spermatozoen handelt.

4. Verwendung gemäß einem der vorhergehenden Ansprüche, wobei es sich bei den numerischen chromosomalen Veränderungen von Gameten um Disomie von Geschlechtschromosomen handelt.

5. Verwendung gemäß einem der vorhergehenden Ansprüche, wobei das männliche Lebewesen ein Mensch ist.

6. Verwendung gemäß einem der vorhergehenden Ansprüche, wobei die Substanz FSH ist.

7. Verwendung gemäß einem der vorhergehenden Ansprüche, wobei die Substanz rFSH ist.

8. Verwendung gemäß einem der vorhergehenden Ansprüche, wobei die Substanz (abwechselnd) jeden zweiten Tag verabreicht wird.

9. Verwendung gemäß einem der vorhergehenden Ansprüche, wobei die Substanz mit oder mit etwa 75 bis 300 IE/Dosis verabreicht wird.

10. Verwendung gemäß einem der vorhergehenden Ansprüche, wobei die Substanz mit oder mit etwa 150 IE/Dosis verabreicht wird.

11. Verwendung gemäß einem der vorhergehenden Ansprüche, wobei die Substanz rFSH ist und der Mann an einer Aneuploidie von Gameten leidet.

## Revendications

1. Emploi d'une substance, choisie parmi l'hormone FSH (folliculostimuline) et ses variants, en vue de la préparation d'une composition pharmaceutique conçue pour traiter et/ou réduire des altérations chromosomiques des gamètes chez un individu mâle.

2. Emploi conforme à la revendication 1, les altérations chromosomiques des gamètes étant des altérations numériques.

3. Emploi conforme à la revendication 1 ou 2, les altérations chromosomiques numériques des gamètes étant une diploïdie des spermatozoïdes.

4. Emploi conforme à l'une des revendications précédentes, les altérations chromosomiques numériques des gamètes étant une disomie de chromosome sexuel.

5. Emploi conforme à l'une des revendications précédentes, l'individu mâle étant un humain.

6. Emploi conforme à l'une des revendications précédentes, dans lequel ladite substance est l'hormone FSH.

7. Emploi conforme à l'une des revendications précédentes, dans lequel ladite substance est une hormone FSH recombinante (rFSH).

8. Emploi conforme à l'une des revendications précédentes, ladite substance étant administrée un jour sur deux.

9. Emploi conforme à l'une des revendications précédentes, ladite substance étant administrée en doses de 75 à 300 UI ou à peu près.

10. Emploi conforme à l'une des revendications précédentes, ladite substance étant administrée en doses de 150 UI ou à peu près.

11. Emploi conforme à l'une des revendications précédentes, dans lequel ladite substance est une hormone rFSH, ledit individu mâle humain souffrant d'aneuploïdie des gamètes.
